(19) **Europäisches Patentamt** / **European Patent Office** / **Office européen des brevets**

(11) **EP 4 393 958 A1**

(12) **EUROPEAN PATENT APPLICATION**
published in accordance with Art. 153(4) EPC

(43) Date of publication:
03.07.2024 Bulletin 2024/27

(21) Application number: 21954735.3

(22) Date of filing: 29.09.2021

(51) International Patent Classification (IPC):
C07K 19/00 (2006.01)　　　C12N 15/62 (2006.01)
C12P 21/02 (2006.01)　　　A61K 38/20 (2006.01)
A61K 39/395 (2006.01)　　　A61P 35/00 (2006.01)

(86) International application number:
PCT/CN2021/121808

(87) International publication number:
WO 2023/024215 (02.03.2023 Gazette 2023/09)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 25.08.2021 CN 202110982310

(71) Applicant: Beijing Vdjbio Co., Ltd.
Beijing 100085 (CN)

(72) Inventors:
• LI, Ziqiang
  Beijing 100085 (CN)
• TIAN, Xinsheng
  Beijing 100085 (CN)
• CHENG, Jianwei
  Beijing 100085 (CN)

• MA, Rong
  Beijing 100085 (CN)
• JIANG, Yanjing
  Beijing 100085 (CN)
• SUN, Yiping
  Beijing 100085 (CN)
• ZHANG, Xiaorui
  Beijing 100085 (CN)
• ZHAO, Tingting
  Beijing 100085 (CN)
• ZHANG, Jun
  Beijing 100085 (CN)

(74) Representative: Dolphin, Kirsty Mairi et al
Venner Shipley LLP
200 Aldersgate
London EC1A 4HD (GB)

Remarks:
The complete document including Reference
Table(s) and the Sequence Listing(s) can be
downloaded from the EPO website

(54) **LONG-ACTING RECOMBINANT HUMAN INTERLEUKIN-2 FUSION PROTEIN, AND PREPARATION METHOD THEREFOR AND APPLICATION THEREOF**

(57) A fusion protein of interleukin-2 and human serum albumin. By electroporating a plasmid carrying interleukin-2 and human serum albumin fusion gene into a CHO cell, a CHO monoclonal cell line stably and efficiently expressing a human recombinant protein is obtained. The monoclonal cell line can secrete and express a fusion protein of interleukin-2 and human serum albumin. The fusion protein can prolong the plasma half-life of human interleukin-2, and can be used for preparing human interleukin-2 drugs or drugs for treating various diseases such as tumors and immunodeficiency diseases.

EP 4 393 958 A1

**Description**

**Technical Field**

The present invention relates to the field of biopharmaceuticals, specifically to a fusion protein of human interleukin-2 and human serum albumin, and coding genes and application thereof.

**Background**

Interleukin-2 (IL-2) is a glycoprotein of 15.5kD produced by T cells and NK cells, which plays an important role in the immune response of bodies. (Human) Interleukin-2 ((h)IL-2) is composed of 133 amino acid residues (SEQ ID NO: 1), with an intra-chain disulfide bond and a cysteine located at the 125th amino acid residue of mature protein. This cysteine is prone to form mismatched disulfide bonds with the other two cysteine residues, and IL-2 with mismatched disulfide bonds is inactive. Mutating the cysteine to serine or alanine can avoid this problem without altering the activity. IL-2 mainly acts on immune cells, including T cells, large granular lymphocytes, monocytes and B cells, promoting cell proliferation and secretion of cytokines. IL-2 has anti-tumor, antimicrobial infection, and immunomodulatory effects *in vivo.* IL-2, either used alone or in combination with IFN-α, monoclonal antibodies, LAK cells, and the like for clinical use, has a certain therapeutic effect on renal cell carcinoma, metastatic melanoma, lymphoma, and the like. It also has therapeutic effects on colon cancer and can improve the tolerance of tumor patients to radiotherapy and chemotherapy. At present, IL-2 is widely used in clinical practice for the treatment of tumors, viral hepatitis, and the like with significant therapeutic effects. However, the above-mentioned diseases generally have a longer course of disease and therefore require long-term use of IL-2. The plasma half-life of IL-2 is only about 2 hours, and high doses and frequent injections are required to maintain its efficacy. This not only greatly increases the cost of treatment, but also increases the patient's pain and side effects.

People extend the half-life of drugs by constructing fusion proteins to increase their molecular weight. Serum albumin is an important component of plasma, and is also a carrier of many endogenous factors and exogenous drugs. Under normal circumstances, it cannot pass through the glomerulus easily. Human serum albumin (HSA) is a protein composed of 585 amino acid residues (SEQ ID NO: 2), with a molecular weight of approximately 66.5KD and a plasma half-life of over 2 weeks.

Patent application No. CN104789594A discloses fed-batch culture of stable cell lines in 3L of shake flask, which was carried out in 80 mL of seed solution having a density of $4.0 \times 10^6$ cells/mL. The cell density reached to $6.4 \times 10^6$ cells/mL on Day 3. After Day 3, the temperature was lowered and the cell density did not change significantly, and the final protein concentration was 118 mg/L. The high expression level of cell line is a basic requirement for the production of biomacromolecules such as antibodies in recent years.

Therefore, there is a need of a fusion protein of human interleukin-2 and serum albumin with higher expression levels and longer efficacy.

**Summary of the Invention**

The objective of the present invention is to provide a fusion protein of human interleukin-2 and serum albumin that can prolong the plasma half-life of human interleukin-2, and the coding gene thereof.

In order to achieve the purpose of the present invention, the present invention provides a long-acting recombinant fusion protein of human interleukin-2 and human serum albumin and a preparation method therefor. The fusion protein of human interleukin-2 and human serum albumin is expressed using a mammalian eukaryotic expression system, and the two are directly connected or connected via a linker peptide.

In the first aspect, the present invention provides an expression system that expresses a fusion protein of human interleukin-2 and human serum albumin, wherein the fusion protein comprises:

a human interleukin-2 or a variant thereof, wherein the human interleukin-2 or a variant thereof comprises: an amino acid sequence set forth in SEQ ID NO: 1; an amino acid sequence set forth in SEQ ID NO: 1 having one or more amino acids substitution, deletion and/or addition and meanwhile retaining equivalent functions; or an amino acid sequence having at least 90% sequence identity to the amino acid sequence set forth in SEQ ID NO: 1; and
a human serum albumin or a variant thereof, wherein the human serum albumin or a variant thereof comprises: an amino acid sequence set forth in SEQ ID NO: 2; an amino acid sequence set forth in SEQ ID NO: 2 having one or more amino acids substitution, deletion and/or addition and meanwhile retaining equivalent functions; or an amino acid sequence having at least 90% sequence identity to the amino acid sequence set forth in SEQ ID NO: 2.

The expression system according to the present invention is a CHO cell; preferably, the expression system is a CHO-K1 cell.

It is unexpectedly discovered in the present invention that when using CHO cells, especially the CHO-K1 cell line, the cell expression level is very high, reaching up to 4g/L; moreover, the expressed fusion protein has a high activity and strongly promotes the proliferation of specific cells such as NK cells and/or T cells *in vitro.*

In some embodiments, the fusion protein of human interleukin-2 and human serum albumin comprises an amino acid sequence set forth in SEQ ID NO: 1 and an amino acid sequence set forth in SEQ ID NO: 2.

The amino acid sequence set forth in SEQ ID NO: 1 is human interleukin-2 composed of 133 amino acid residues, and the amino acid sequence set forth in SEQ ID NO: 2 is human serum albumin composed of 585 amino acid residues.

In some embodiments, the amino acid at position 125 of SEQ ID NO: 1 in the fusion protein is not cysteine, and preferably, the amino acid at position 125 is substituted with serine or alanine.

In some embodiments, the human interleukin-2 or a variant thereof is directly connected to the human serum albumin or a variant thereof, or the human interleukin-2 or a variant thereof is connected to the human serum albumin or a variant thereof via a linker peptide.

In the fusion protein according to the present invention, in order to create a large gap between the two moieties contained in the fusion protein and maximize the binding of the human interleukin-2 moiety to the interleukin-2 receptor, a linker peptide is provided between the human interleukin-2 or a variant thereof and the human serum albumin or a variant thereof. Preferably, the linker peptide is represented by a general formula $(G_nS)_m$, wherein n and m are an integer from 1 to 10, respectively; more preferably, n is an integer from 1 to 4, and m is an integer from 0 to 3.

In some embodiments, the N-terminus of the fusion protein carries a signal peptide; preferably, the signal peptide is a secretary signal peptide CD33; more preferably, the signal peptide sequence is MPLLLLLPLLWAGALA, as set forth in SEQ ID NO: 5.

In some embodiments, the fusion protein is encoded by the nucleotide sequence set forth in SEQ ID NO: 3.

In the present invention, based on the gene sequences of human interleukin-2 and human serum albumin published by NCBI, gene sequence optimization is carried out according to mammalian codon preference. The optimized gene sequence of a fusion protein is constructed onto an expression vector, transfected into a host cell, expressed in the host cell, isolated and purified to obtain a target protein.

The optimized gene sequence of the fusion protein according to the present invention is added with a signal peptide at its N-terminus, preferably added with a secretary signal peptide CD33 sequence, constructed onto an expression vector, and the obtained vector is transfected into a host cell, expressed in the host cell, isolated and purified to provide a target protein.

In the present invention, the optimized gene sequence carries a signal peptide at its N-terminus, and the gene sequence that is constructed onto the expression vector is set forth in SEQ ID NO: 3.

In some embodiments, the fusion protein of human interleukin-2 and human serum albumin has an amino acid sequence set forth in SEQ ID NO: 4.

Wherein, SEQ ID NO: 4 is a protein composed of 728 amino acid residues. Position 1 from the N-terminus to position 133 correspond to a coding sequence for human interleukin-2, position 134 from the N-terminus to position 143 correspond to a coding sequence for a linker peptide Gly Gly Gly Gly Ser Gly Gly Gly Gly Ser, and position 144 from the N-terminus to position 728 correspond to a coding sequence for human serum albumin.

In the second aspect, the present invention provides the use of the expression system in the preparation of a medicament for the treatment of tumors, hepatitis, pneumonia, or immunodeficiency diseases.

**Beneficial effects of the present invention**

The present invention discloses an expression system expressing a fusion protein of interleukin-2 and human serum albumin. By electroporation of the plasmid with the fusion gene of interleukin 2 and human serum albumin into CHO cells, a CHO monoclonal cell line with stable and efficient expression of human recombinant protein was obtained. The monoclonal cell line according to the present invention can secrete a fusion protein expressing interleukin-2 and human serum albumin. This fusion protein can prolong the plasma half-life of human interleukin-2 and can be used to prepare human interleukin-2 drugs or drugs for treating various diseases such as tumors and immunodeficiency diseases.

The present invention achieves high expression levels not only through clone screening, but also by adding a signal peptide to N-terminus of the optimized fusion protein gene sequence during cell construction, which is also one of the reasons for high expression levels.

The cell line of the present invention not only has a high expression level of up to 4 g/L, but also expresses fusion proteins with high activity and having a strong promoting effect on proliferation of NK cells and/or T cells.

**Description of Drawings**

Figure 1 shows reductive electrophoresis analysis results of a well plate culture supernatant of IL-2-HSA/CHOK1 clone screening, with 20 $\mu$L of supernatant sample added.

Figure 2 shows non-reductive electrophoresis analysis results of the fed-batch culture D13 supernatant of IL-2-HSA/CHOK1 clone screening, with 5 $\mu$L of supernatant sample added.

Figure 3 shows non-reductive electrophoresis analysis results of the fed-batch culture D13 supernatant of IL-2-HSA/CHOK1 clone #9 subjected to monoclonal screening, with 3 $\mu$L of supernatant sample added.

Figure 4 shows non-reductive electrophoresis analysis results of the fed-batch culture D13 supernatant of IL-2-HSA/CHOK1 clone #9-6 subjected to monoclonal screening, with 2 $\mu$L of supernatant sample added.

Figure 5 shows a kinetics curve of IL-2-HSA/CHOK1 cell culture. Fed-batch culturing was carried out in a 125 mL shake flask with an initial cell density of $0.3\times10^6$ cells/mL, an initial culture volume of 25 mL, and a maximum live cell density of $19.3\times10^6$ cells/mL.

Figure 6 shows a protein expression kinetics curve of the supernatant D7-D13 obtained from IL-2-HSA/CHOK1 cells through low-density inoculation culture process.

Figure 7 shows non-reductive electrophoresis analysis results of IL-2-HSA/CHOK1 cell expression supernatant, with 1 $\mu$L of supernatant sample added.

Figure 8 shows a kinetics curve of IL-2-HSA/CHOK1 cell culture. Fed-batch culture was carried out in a 1 L shake flask with an initial cell density of $2\times10^6$ cells/mL, an initial culture volume of 300 mL and a maximum live cell density of $16\times10^6$ cells/mL.

Figure 9 shows a protein expression kinetics curve of the supernatant D0-D10 obtained from IL-2-HSA/CHOK1 cells through a high-density inoculation culture process.

Figure 10 shows non-reductive electrophoresis analysis results of IL-2-HSA/CHOK1 cell expression supernatant, with 5 $\mu$L of supernatant sample added.

Figure 11 shows a curve of NK-92 proliferation stimulated by IL-2-HSA.

Figure 12 shows a curve of CTLL-2 proliferation stimulated by IL-2-HSA.

**Best Mode of the Invention**

The present invention is further illustrated through the examples below. It should be understood that the examples of the present invention are only intended to illustrate the present invention but not to limit it. Any simple improvement made to the present invention under the concept of the present invention is within the scope of protection claimed by the present invention.

**Example 1: Construction of Stable Transfected Cell Lines**

On the day before transfection, the density of CHO-K1 cells was adjusted to $0.5\times10^6$ cells/mL. On the day of transfection, high concentration endotoxin-free plasmids that had undergone linear treatment were prepared, the cell density and viability of CHO-K1 cells were measured, ensuring that the cell viability was greater than 97%. After the CHO-K1 cells were washed twice with CD CHO medium, an electroporation transfection reaction system was prepared: 700 $\mu$L of cell suspension and 40 $\mu$g of plasmid were mixed well and transferred into a 4 mm of electrode cup. The electrode cup was placed into an electroporator and the shock parameters were set to 300 V, 1000 $\mu$F. After one electric shock, the shocked cell suspension was transfer into preheated fresh CD CHO medium, and incubated at 37 °C for 20 minutes. The incubated cell suspension was inoculated evenly into a 96-well plate, transfected for 24 hours, pressurized, and added with CD CHO medium containing methionine sulfoximine (MSX), under the final screened pressure of 25-50 $\mu$M MSX, with 5% $CO_2$, at 37 °C for static culture.

**Example 2: Screening of High Expression Monoclonal Cell Lines**

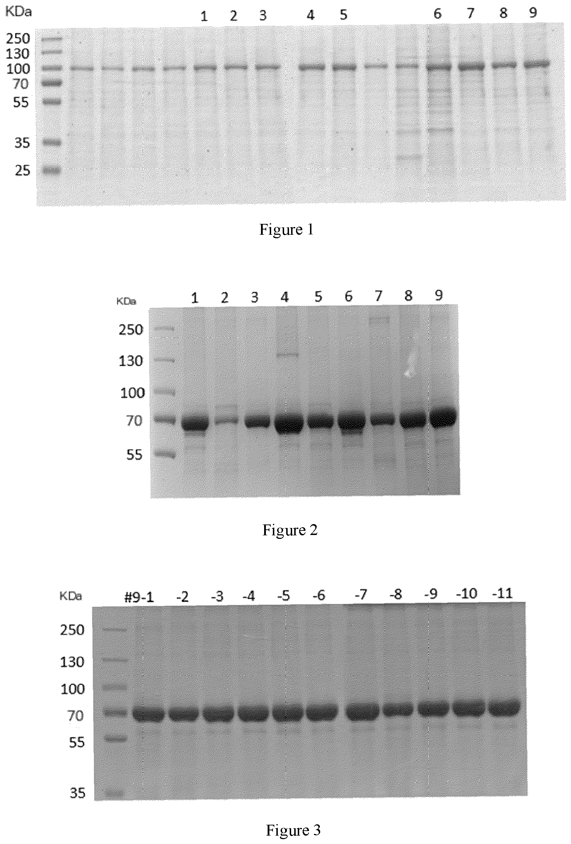

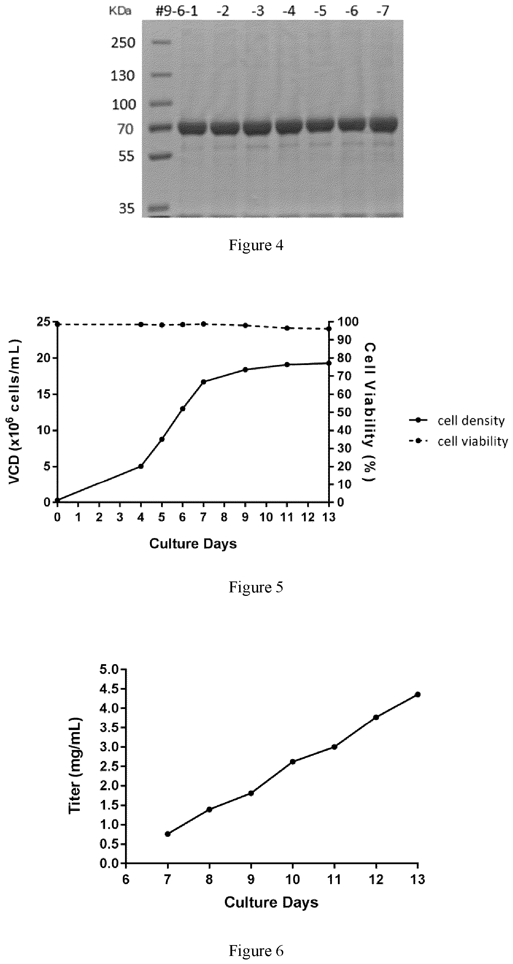

After growing to an appropriate size in the 96-well plate, the monoclonal cell lines were selected and all clones were transferred to a new 96-well plate, incubated in 5% $CO_2$ at 37 °C for static culture. After the cells in the well were fully grown, the supernatant was taken from the well plate for reductive electrophoresis to detect the expression of fusion proteins. Nine clones with the highest expression levels were selected (see Figure 1) and gradually expanded to shake flask culture. Nine clones were subjected to fed-batch culture in 25 mL shake flasks, and the culture supernatant was harvested and identified by non-reductive electrophoresis (see Figure 2). The cell line # 9 with the best expression level was selected. Monoclonal cell lines were screened using limited dilution method for cell line # 9. A 96-well plate was inoculated with 0.3 cells/well to obtain 11 highly expressed cell lines, which were subjected to fed-batch culture in 25 mL shake flasks, and the supernatant was identified by non-reductive electrophoresis (see Figure 3). The cell line #9-6 with the best expression was selected. Monoclonal cell lines were screened again using limited dilution method for cell line #9-6, resulting in 7 high expressed cell lines, which were subject to fed-batch culture in 25 mL shake flasks and the supernatant was identified by non-reductive electrophoresis (see Figure 4). Cell line #9-6-7 with good stability and high expression levels was selected as stable and high expression cell line IL-2-HSA/CHOK1.

**Example 3: Fed-Batch Culture of Stable Cell Line in 125 mL Shake Flask**

On the day (recorded as D0) when IL-2-HSA/CHOK1 cells were started to be cultured for expression, 25 mL of minimum medium containing 25-50 $\mu$M MSX were inoculated into a 125 mL of shake flask at a density of $0.3 \times 10^6$ cells/mL, and cultured with 5% $CO_2$ at 37 °C on a shaker at 135 rpm. On D4 of inoculation, the samples were taken and counted, and the culture temperature was decreased to 33 °C when the cell density reached to $10 \times 10^6$ cells/mL. On D5, fed-batch culture was carried out and the glucose concentration was controlled to 3 ~ 4 g/L. On D13 of culture, the culture was terminated, the supernatant of the cell culture solution was collected, and the expression level of the fusion protein was measured to be 4.36 mg/mL.

A kinetics curve of IL-2-HSA/CHOK1 cell culture is shown in Figure 5. It can be seen from Figure 5 that in the early stage of culture, the cells were in a logarithmic growth stage, with a rapid increase in density; in the later stage of culture, the cells entered a protein production stage, and the cell density tended to stabilize. The maximum live cell density reached to $19.3 \times 10^6$ cells/mL.

A kinetics curve of IL-2-HSA/CHOK1 cell supernatant expression level is shown in Figure 6. It can be seen from Figure 6 that from D7 to D13, with the increase of culture days, the protein expression level of the cell showed a gradually increasing trend. The expression level on D13 was 4.36 mg/mL.

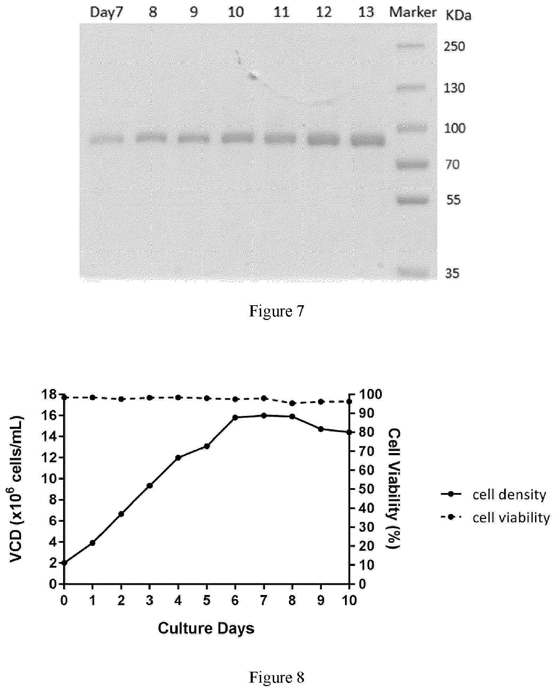

A non-reductive electrophoresis analysis of IL-2-HSA/CHOK1 cell expression supernatant is shown in Figure 7. It can be seen from Figure 7 that from D7 to D13, with the increase of culture days, the protein expression level of the cell showed a gradually increasing trend. The target protein had a single band without impurities.

**Example 4: Fed-Batch Culture of Stable Cell Line in 1 L of Shake Flask**

IL-2-HSA/CHOK1 cells were inoculated into a 50 mL of shaking flask at a density of $2 \times 10^6$ cells/mL, and cultured with 5% $CO_2$ at 37 °C on a shaker at 135 rpm. The samples were taken and counted daily to observe cell status, and the minimum medium containing 25-50 $\mu$M MSX was supplemented, with daily adjustment of cell density to $2 \times 10^6$ cells/mL until the volume of the cell culture solution reached to 300 mL. The minimum medium was stopped supplementing, and the culture was continued, at which point it was recorded as D0. The samples were taken and counted daily and 1 mL of culture supernatant was retained. The culture temperature was decreased to 33 °C when the cell density reached to $6 \times 10^6$ ~ $7 \times 10^6$ cells/mL. On D2, the fed-batch culture was started and the glucose concentration was controlled to 3 g/L. On D10 of culture, the culture was terminated, the supernatant of the cell culture solution was collected, and the protein expression level of the supernatant was measured from D0 to D10. The expression level of fusion protein in the supernatant harvested on D10 was measured to be 3.12 mg/mL.

A kinetics curve of IL-2-HSA/CHOK1 cell culture is shown in Figure 8. It can be seen from Figure 8 that in the early stage of culture, the cells were in a logarithmic growth stage, with a rapid increase in density; in the later stage of culture, the cells entered a protein production stage, and the cell density tended to stabilize. The maximum live cell density reached to $16 \times 10^6$ cells/mL.

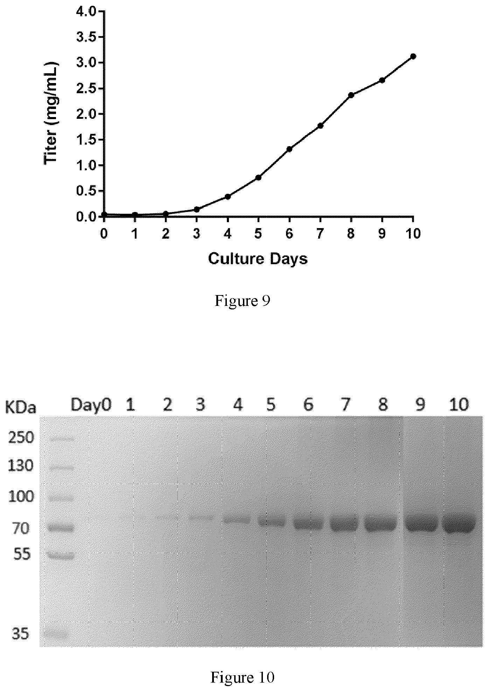

A kinetics curve of IL-2-HSA/CHOK1 cell supernatant expression level is shown in Figure 9. It can be seen from Figure 9 that with the increase of culture days, the protein expression level of the cell showed a gradually increasing trend.

A non-reductive electrophoresis analysis of IL-2-HSA/CHOK1 cell expression supernatant is shown in Figure 10. It can be seen from Figure 10 that with the increase of culture days, the protein expression level of the cell showed a gradually increasing trend. The target protein had a single band and no impurities.

Compared with the fed-batch culture in shake flask in the prior art, the cell density and cell survival rate of the present invention are very high, as shown in Figure 8. The cell density reaches to $14 \times 10^6$ ~ $16 \times 10^6$ cells/mL on D6 to D10, and the cell protein expression level of the present invention is also very high, as shown in Figure 9. The expression level on D10 is 3.12 mg/mL. In the present application, when the cell density is $14 \times 10^6$ ~ $16 \times 10^6$ cells/mL, the expression level is also high, indicating that the cell activity is still good at such a cell density.

**Example 5: Activity Experiment 1 of Fusion Protein: Detection of Effect of IL-2-HAS on NK-92 Cell Proliferation**

In this example, NK-92 cells (ATCC® CRL-2407 ™, IL-2 dependent NK cell line derived from peripheral blood monocytes of a 50 year-old male patient with malignant non-Hodgkin's lymphoma) were utilized to evaluate the biological activity of IL-2-HSA.

(1) Cryopreserved NK-92 cells were taken out from liquid nitrogen tank and resuscitated for culture until the logarithmic growth phase.

(2) Sufficient cells were collected by centrifugation, resuspended in complete culture medium without IL-2, and cultured under starvation for 24 hours.

(3) The starved NK-92 cells were centrifuged, resuspend in complete culture medium without IL-2, and counted; the cell density was adjusted to $5 \times 10^5$ cells/mL, and the cells were added into the 96-well plate at a volume of 90 $\mu$L per well.

(4) Preparation of sample solution: IL-2-HSA sample and rhIL-2 (R&D, Cat. No. 202-IL) were pre-diluted to 50.67 nM with the medium, and diluted to 9 concentrations at a 4-fold gradient, added to corresponding wells of the 96-well plate at 10 $\mu$L/well, with three wells for each concentration. For the negative control group, the medium was added at 10 $\mu$L/well correspondingly and mixed well.

(5) After culturing in 5% $CO_2$ at 37 °C for 72 hours, the melted and mixed MTS detection reagent was added to the above 96-well plate at 20 $\mu$L/well, shook well in an oscillator, and then incubated in a 37 °C, 5% $CO_2$ cell culture incubator for 1 to 4 hours.

(6) After incubation, shook and mixed well; the absorbance was measured at a wavelength of 490 nm using a microplate reader.

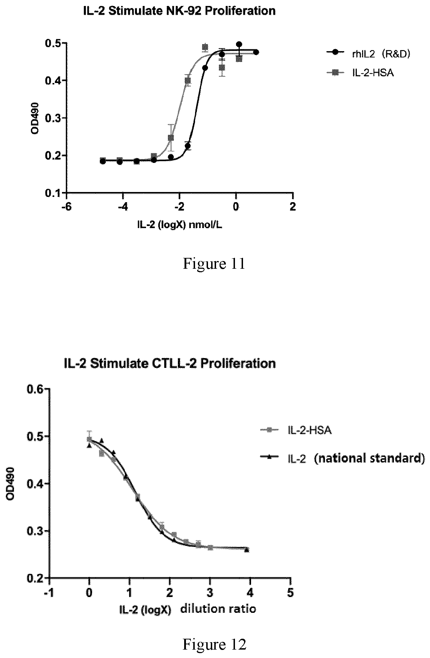

(7) The data was analyzed using GraphPad Prism 8 software, with the logarithm of drug concentration X as the horizontal coordinate and $OD_{490}$ as the vertical coordinate. The drug action curve was fitted with four parameters. The $EC_{50}$ values obtained are shown in Table 1, and the proliferation curve is shown in Figure 11.

Table 1

|  | IL-2 (R&D) | IL-2-HSA |
|---|---|---|
| $EC_{50}$ (nM) | 0.0420 | 0.01047 |

It can be seen from Figure 11 that IL-2-HSA induced NK-92 cell growth in a dose-dependent manner. Under the experimental condition, the activity of IL-2-HSA in stimulating NK-92 proliferation was better than that of equimolar rhIL-2 (about 4 times).

**Example 6: Activity Experiment 2 of Fusion Protein: Detection of Effect of IL-2-HAS on CTLL-2 Cell Proliferation**

In this example, CTLL-2 cells (ATCC® TIB™, mouse cytotoxic T lymphocyte cell line, IL-2 dependent) were utilized to evaluate the biological activity of IL-2-HSA.

Referring to the human interleukin-2 biological activity assay method (CTLL-2 cell/MTT colorimetric method) in the Chinese Pharmacopoeia, CTLL-2 cells were inoculated into 96-well plate at a density of 30000 cells/well. A series of gradient diluted national standards and IL-2-HSA were added, and cultured in 5% $CO_2$ at 37 °C for 18 to 24 hours. Then, MTS detection reagent was added and incubated for 1 to 4 hours. After shaking and mixing, the absorbance was measured at a wavelength of 490 nm using a microplate reader. The data was analyzed using GraphPad Prism 8 software, with the logarithm of dilution X as the horizontal coordinate and $OD_{490}$ as the vertical coordinate. The drug action curve was fitted with four parameters. The $EC_{50}$ values obtained are shown in Table 2, and the proliferation curve is shown in Figure 12.

The biological activity of IL-2-HSA is calculated using the following formula:

$$\text{biological activity of test sample} \frac{\text{IU}}{\text{mL}}$$
$$= \text{biological activity of standard IU/mL} \times \frac{\text{pre} - \text{dilution ratio of test sample}}{\text{pre} - \text{dilution ratio of standard}}$$
$$\times \frac{\text{dilution ratio of test sample equivalent to the half effective amount of standard}}{\text{semi} - \text{effective dilution ratio of standard}}$$

After calculation, the specific activity of IL-2-HSA was $8.38 \times 10^6$ IU/mg, comparable to the specific activity (not less than $1 \times 10^7$ IU/mg) of IL-2 as specified in the pharmacopoeia, but the molecular weight difference between the two was about 5 times, thus the specific activity of IL-2-HSA was higher.

Table 2

| | IL-2 (National Standard) | IL-2-HSA |
|---|---|---|
| $EC_{50}$ (dilution ratio) | 13.9 | 11.68 |

The examples listed in the present invention are only the preferred technical solutions of the present invention. The scope of protection of the present invention is not limited to the above-mentioned examples. If any modification or deformation is carried out under the principal conditions of the present invention, it should belong to the scope of protection of the present invention.

**Claims**

1. An expression system **characterized in that** the expression system expresses a fusion protein of human interleukin-2 and human serum albumin, wherein the fusion protein comprises:

    a human interleukin-2 or a variant thereof, wherein the human interleukin-2 or a variant thereof comprises: an amino acid sequence set forth in SEQ ID NO: 1; an amino acid sequence set forth in SEQ ID NO: 1 having one or more amino acids substitution, deletion and/or addition and meanwhile retaining equivalent functions; or an amino acid sequence having at least 90% sequence identity to the amino acid sequence set forth in SEQ ID NO: 1; and
    a human serum albumin or a variant thereof, wherein the human serum albumin or a variant thereof comprises: an amino acid sequence set forth in SEQ ID NO: 2; an amino acid sequence set forth in SEQ ID NO: 2 having one or more amino acids substitution, deletion and/or addition and meanwhile retaining equivalent functions; or an amino acid sequence having at least 90% sequence identity to the amino acid sequence set forth in SEQ ID NO: 2.

2. The expression system according to claim 1, **characterized in that** the expression system is a CHO cell; preferably, the expression system is a CHO-K1 cell.

3. The expression system according to claim 1, **characterized in that** the fusion protein of human interleukin-2 and human serum albumin comprises an amino acid sequence set forth in SEQ ID NO: 1 and an amino acid sequence set forth in SEQ ID NO: 2.

4. The expression system according to claim 3, **characterized in that** the amino acid at position 125 of SEQ ID NO: 1 in the fusion protein is not cysteine; preferably, the amino acid at position 125 is substituted with serine or alanine.

5. The expression system according to any one of claims 1 to 4, **characterized in that** the human interleukin-2 or a variant thereof is directly connected to the human serum albumin or a variant thereof, or the human interleukin-2 or a variant thereof is connected to the human serum albumin or a variant thereof via a linker peptide,
preferably, the linker peptide is represented by a general formula $(G_nS)_m$, wherein n and m are an integer from 1 to 10, respectively; more preferably, n is an integer from 1 to 4, and m is an integer from 0 to 3.

6. An expression system according to any one of claims 1 to 4, **characterized in that** the N-terminus of the fusion protein carries a signal peptide;
preferably, the signal peptide is secretory signal peptide CD33.

7. The expression system according to claim 6, **characterized in that** the signal peptide has a sequence set forth in SEQ ID NO: 5.

8. The expression system according to claim 1, **characterized in that** the fusion protein is encoded by a nucleotide sequence set forth in SEQ ID NO: 3.

9. The expression system according to claim 1, **characterized in that** the fusion protein of human interleukin-2 and human serum albumin has an amino acid sequence set forth in SEQ ID NO: 4.

10. Use of the expression system according to any one of claims 1 to 9 in the preparation of a medicament for the treatment of tumors, hepatitis, pneumonia, or immunodeficiency diseases.

Figure 1

Figure 2

Figure 3

Figure 4

Figure 5

Figure 6

Figure 7

Figure 8

Figure 9

Figure 10

**IL-2 Stimulate NK-92 Proliferation**

Figure 11

**IL-2 Stimulate CTLL-2 Proliferation**

Figure 12

## INTERNATIONAL SEARCH REPORT

| | International application No. |
|---|---|
| | **PCT/CN2021/121808** |

**A. CLASSIFICATION OF SUBJECT MATTER**

C07K 19/00(2006.01)i; C12N 15/62(2006.01)i; C12P 21/02(2006.01)i; A61K 38/20(2006.01)i; A61K 39/395(2006.01)i; A61P 35/00(2006.01)i

According to International Patent Classification (IPC) or to both national classification and IPC

**B. FIELDS SEARCHED**

Minimum documentation searched (classification system followed by classification symbols)

C07K; C12N; C12P; A61K; A61P

Documentation searched other than minimum documentation to the extent that such documents are included in the fields searched

Electronic data base consulted during the international search (name of data base and, where practicable, search terms used)

CNABS; VEN; CNTXT; CJFD; ENTXTC; CNKI; 万方, WANFANG; ISI WEB OF KNOWLEDGE; NCBI; STN: 白细胞介素-2, 白介素2, HSA, 人血清白蛋白, IL-2, interleukin, serum albumin, fusion protein, 融合, 半衰期, 长效, 连接肽, 信号肽, 仓鼠卵巢细胞, CHO, SEQ ID NOs: 1-5

**C. DOCUMENTS CONSIDERED TO BE RELEVANT**

| Category* | Citation of document, with indication, where appropriate, of the relevant passages | Relevant to claim No. |
|---|---|---|
| X | CN 112724259 A (TIANJIN LINDA BIOTECHNOLOGY CO., LTD. et al.) 30 April 2021 (2021-04-30)<br>  claims 1-10, and sequence table | 1-10 |
| X | CN 104789594 A (JIANGNAN UNIVERSITY) 22 July 2015 (2015-07-22)<br>  claims 1-7 | 1-10 |
| X | US 2017204154 A1 (DELINIA, INC.) 20 July 2017 (2017-07-20)<br>  SEQ ID NO.25 | 1-10 |
| X | CN 1626554 A (INSTITUTE OF BIOTECHNOLOGY, THE ACADEMY OF MILITARY MEDICAL SCIENCES, CHINA) 15 June 2005 (2005-06-15)<br>  claims 1-10 | 1-10 |
| X | CN 101280018 A (JIANGNAN UNIVERSITY) 08 October 2008 (2008-10-08)<br>  claims 1-10 | 1-10 |
| X | CN 1884520 A (JIANGNAN UNIVERSITY) 27 December 2006 (2006-12-27)<br>  claims 1-10 | 1-10 |

☑ Further documents are listed in the continuation of Box C.  ☑ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **07 May 2022** | **19 May 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

## INTERNATIONAL SEARCH REPORT

| International application No. |
|---|
| **PCT/CN2021/121808** |

**C.    DOCUMENTS CONSIDERED TO BE RELEVANT**

| Category* | Citation of document, with indication, where appropriate, of the relevant passages | Relevant to claim No. |
|---|---|---|
| X | 卢慧惠等 (LU, Huihui et al.). "人血清白蛋白-白介素-2融合蛋白在CHO细胞中的表达 (Expression of Human Serum Albumin-Interleukin-2 Fusion Protein in Chinese Hamster Ovary Cells)" 食品与生物技术学报 *(Journal of Food Science and Biotechnology)*, Vol. 36, No. 5, 15 May 2017 (2017-05-15), pp. 456-460 | 1-10 |
| X | LEI, Jianyong et al. "Expression, purification and characterization of recombinant human interleukin-2-serum albumin (rhIL-2-HSA) fusion protein in Pichia pastoris" *Protein Expression and Purification,* Vol. 84, 17 May 2012 (2012-05-17), pp. 154-160 | 1-10 |
| A | WO 2014139468 A1 (ADMARK HEALTHCARE, LLC) 18 September 2014 (2014-09-18) entire document | 1-10 |

**INTERNATIONAL SEARCH REPORT**

| International application No. |
| --- |
| **PCT/CN2021/121808** |

**Box No. I    Nucleotide and/or amino acid sequence(s) (Continuation of item 1.c of the first sheet)**

1.  With regard to any nucleotide and/or amino acid sequence disclosed in the international application, the international search was carried out on the basis of a sequence listing:

    a.  ☑ forming part of the international application as filed:

        ☑ in the form of an Annex C/ST.25 text file.

        ☐ on paper or in the form of an image file.

    b.  ☐ furnished together with the international application under PCT Rule 13*ter*.1(a) for the purposes of international search only in the form of an Annex C/ST.25 text file.

    c.  ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2.  ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3.  Additional comments:

Form PCT/ISA/210 (continuation of first sheet) (January 2015)

## INTERNATIONAL SEARCH REPORT
### Information on patent family members

International application No.

**PCT/CN2021/121808**

| Patent document cited in search report | | | Publication date (day/month/year) | Patent family member(s) | | | Publication date (day/month/year) |
|---|---|---|---|---|---|---|---|
| CN | 112724259 | A | 30 April 2021 | None | | | |
| CN | 104789594 | A | 22 July 2015 | None | | | |
| US | 2017204154 | A1 | 20 July 2017 | US | 2019202881 | A1 | 04 July 2019 |
| | | | | US | 2019153058 | A1 | 23 May 2019 |
| | | | | US | 2021070828 | A1 | 11 March 2021 |
| | | | | US | 2019202882 | A1 | 04 July 2019 |
| | | | | AU | 2017210187 | A1 | 19 July 2018 |
| | | | | EA | 201891656 | A1 | 28 December 2018 |
| | | | | CL | 2018001951 | A1 | 28 December 2018 |
| | | | | CO | 2018008558 | A2 | 21 August 2018 |
| | | | | SG | 11201805784 P | A | 30 August 2018 |
| | | | | BR | 112018014671 | A2 | 11 December 2018 |
| | | | | US | 2018037624 | A1 | 08 February 2018 |
| | | | | US | 2020115429 | A1 | 16 April 2020 |
| | | | | EP | 3405482 | A1 | 28 November 2018 |
| | | | | EC | SP18062614 | A | 31 October 2018 |
| | | | | CA | 3010621 | A1 | 27 July 2017 |
| | | | | JP | 2019507589 | A | 22 March 2019 |
| | | | | KR | 20180100237 | A | 07 September 2018 |
| | | | | MX | 2018008840 | A | 09 November 2018 |
| | | | | ZA | 201804458 | B | 25 September 2019 |
| | | | | AU | 2021257949 | A1 | 25 November 2021 |
| | | | | WO | 2017127514 | A1 | 27 July 2017 |
| | | | | CN | 108602871 | A | 28 September 2018 |
| CN | 1626554 | A | 15 June 2005 | None | | | |
| CN | 101280018 | A | 08 October 2008 | None | | | |
| CN | 1884520 | A | 27 December 2006 | None | | | |
| WO | 2014139468 | A1 | 18 September 2014 | CN | 105518143 | A | 20 April 2016 |

Form PCT/ISA/210 (patent family annex) (January 2015)

**REFERENCES CITED IN THE DESCRIPTION**

**Patent documents cited in the description**

- CN 104789594 A